# EUROPÄISCHE PATENTSCHRIFT

(11) **EP 1 874 422 B1**
(45) Veröffentlichungstag und Bekanntmachung des Hinweises auf die Patenterteilung: **08.10.2008**
(21) Anmeldenummer: 06754772.9
(22) Anmeldetag: 20.04.2006
(51) Int. Cl.: B01D 11/02, B01D 9/00

(54) **VERFAHREN ZUR REGELUNG EINER HYDRAULISCHEN WASCHKOLONNE**
METHOD FOR THE REGULATION OF A HYDRAULIC WASH COLUMN
PROCEDE DE REGULATION D'UNE COLONNE DE LAVAGE HYDRAULIQUE

(30) Priorität: 21.04.2005 DE 102005018702; 21.04.2005 US 673339 P
(43) Veröffentlichungstag der Anmeldung: 09.01.2008
(73) Patentinhaber: BASF SE, 67056 Ludwigshafen (DE)
(72) Erfinder: HEILEK, Jörg, 69245 Bammental (DE); MÜLLER-ENGEL, Klaus Joachim, 76297 Stutensee (DE); HAMMON, Ulrich, 68163 Mannheim (DE); WALTER, Thomas, 67454 Hassloch (DE)
(86) Internationale Anmeldenummer: PCT/EP2006/061714
(87) Internationale Veröffentlichungsnummer: WO 2006/111565

(56) Entgegenhaltungen:
- WO-A-02/09839
- US-A- 3 267 686
- US-A- 3 628 341

## Beschreibung

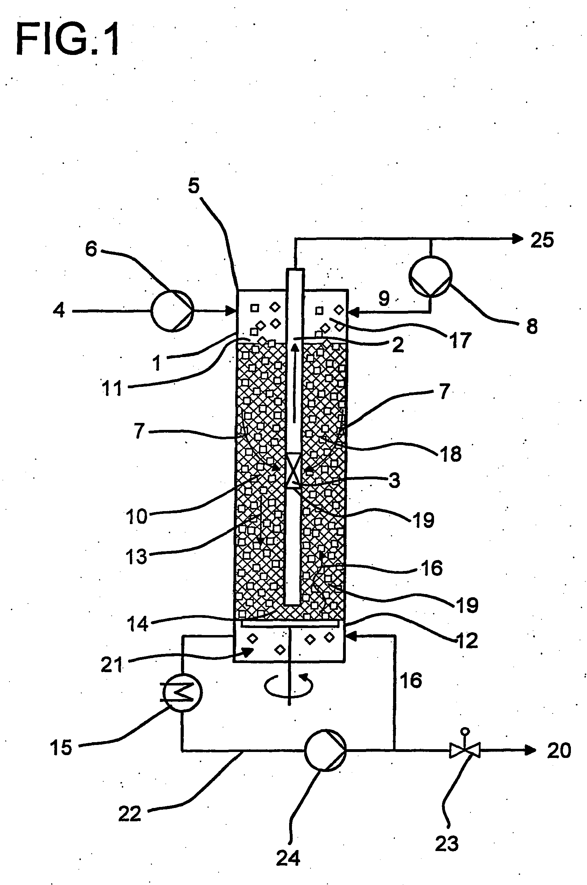

Vorliegende Erfindung betrifft ein Verfahren zur Regelung einer hydraulischen Waschkolonne (vgl. Fig. 1, auf die sich die folgenden numerischen Adressen und die numerischen Adressen in den Patentansprüchen beziehen), die eine zylindrische Einhüllende (1) aufweist, die die Kolonne begrenzt und in der sich parallel zur Zylinderachse ein oder mehrere Filterrohre (2) durch die Kolonne erstrecken, die in der Nähe des zweiten Endes der Kolonne in der Filterrohrwand wenigstens ein Filter (3) aufweisen, das die einzige direkte Verbindung zwischen dem unter dem Druck P1 stehenden Filterrohrinneren und dem Inneren der Kolonne bildet, bei dem man
- wenigstens einen Strom einer Suspension (4), die in einer Mutterlauge suspendierte Kristalle einer zu reinigenden (einer möglichst rein abzutrennenden) Substanz enthält, am ersten Ende der Kolonne (5) mit einem Druck P2 (z.B. mittels einer Pumpe (6)), der größer als P1 ist, in selbige- kontinuierlich einspeist,
- über die Filter Mutterlauge (7) in das Filterrohrinnere hinein und über die Filter rohre aus der Kolonne herausführt,
- am ersten Ende der Kolonne und/oder zwischen diesem Ende und dem Filterbeginn gegebenenfalls Steuerlauge (9) in die Waschkolonne führt,
- durch die Mutter- und gegebenenfalls Steuerlaugeführung in der Kolonne ein Kristallbett (10) der zu reinigenden Substanz ausbildet, das eine dem ersten Ende der Kolonne zugewandte Aufbaufront (11) aufweist, an der sich kontinuierlich Kristalle der eingeleiteten Suspension ans Kristallbett anlagern,
- das Kristallbett durch die aus dem hydraulischen Strömungsdruckverlust der Mutter- und gegebenenfalls Steuerlaugeführung in der Kolonne resultierende Kraft an den Filtern vorbei in die zwischen den Filtern und dem zweiten Ende der Waschkolonne (12) befindliche Waschzone transportiert (13),
- an dem der Aufbaufront gegenüberliegenden Ende des Kristallbetts kontinuierlich Kristalle abträgt (14),
- die abgetragenen Kristalle aufschmilzt (15) und einen Teil der Schmelze als Waschflüssigkeitsstrom vom zweiten Ende der Kolonne herkommend gegen die Transportrichtung der Kristalle durch das Kristallbett leitet (16), und
- die Position (Lage) der Aufbaufront mit Hilfe des Mengenstroms von in die Waschkolonne geführter Steuerlauge und/oder mit Hilfe des Mengenstroms.der in die Waschkolonne geführten Suspension (4) regelt.

Im besonderen betrifft vorliegende Erfindung die Regelung einer hydraulischen Waschkolonne, die zum reinigenden Abtrennen von Acrylsäurekristallen aus ihrer Suspension in verunreinigter Acrylsäure betrieben wird, wie es in den Schriften WO 01/77056, WO 04/35514, WO 03/41833, WO 02/9839, WO 03/41832, DE-A 100 36 881, WO 02/55469 und WO 03/78378 beschrieben ist. Die numerischen Adressen in dieser Schrift beziehen sich stets auf die dieser Schrift beiliegenden Figuren.

Die Begriffe zylindrisch und rohrförmig sollen in dieser Schrift so verstanden werden, dass sie alle geometrischen Formen (Körper) umfassen, deren Querschnitt kreisförmig oder kreisähnlich (z.B. eliptisch oder vieleckig (z.B. regelmäßiges Viereck, Sechseck oder Achteck) ist.

Der Begriff Mutterlauge soll in dieser Schrift so verstanden werden, dass er insbesondere Schmelzen aus der zu reinigenden Substanz und Verunreinigungen und/oder aber auch Lösungen aus der zu reinigenden Substanz und Lösungsmitteln bzw. Lösungsmittelgemischen sowie Verunreinigungen umfasst. Ebenso soll der Begriff "Schmelze von abgetragenen Kristallen als Waschflüssigkeitsstrom" vorzugsweise Schmelze von abgetragenen Kristallen, aber auch gesättigte Lösungen von abgetragenen Kristallen in Lösungsmitteln bzw. Lösungsmittelgemischen umfassen. Entsprechend umfasst "aufschmelzen von abgetragenen Kristallen" auch "sättigendes lösen von abgetragenen Kristallen in Lösungsmitteln bzw. Lösungsmittelgemischen". Acrylsäure, entweder für sich oder in Form ihrer Salze oder ihrer Ester, ist insbesondere zur Herstellung von Polymerisaten für die verschiedensten Anwendungsgebiete (z.B. Klebstoffe, Superabsorber, Bindemittel) von Bedeutung.

Das Verfahren gemäß der Präambel dieser Schrift ist bekannt (vgl. z.B. EP-A 097 405, WO 03/041832, DE-A 100 36 881, WO 02/09839, WO 03/041833, WO 01/77056 und WO 03/063997).

Es schließt sich in der Regel an eine Suspensionskristallisation an, die ein sehr wirksames und kostengünstiges Verfahren bildet, um eine hohe Reinheit einer gewünschten chemischen Verbindung (einer chemischen Substanz) zu erzielen.

Bei der Synthese einer chemischen Verbindung fällt die gewünschte Substanz üblicherweise nämlich nicht als Reinprodukt, sondern als Teil eines Verbindungsgemisches an, das neben der gewünschten Substanz Verunreinigungen wie nicht umgesetzte Ausgangsverbindungen, Lösungsmittel, Nebenprodukte oder unerwünschte Isomere enthält.

Handelt es sich bei der gewünschten Substanz um eine kristallisierbare Verbindung (z.B. Acrylsäure), die nach dem Syntheseprozess in einem flüssigen Verbindungsgemisch vorliegt oder in ein solches überführt werden kann (vgl.z.B. EP-A 1 015 411, DE-A 196 06 877, DE-A 103 36 386, EP-A 792 867, DE-A 102 35 847, WO 03/078378, WO 02/055469 im Fall von Acrylsäure bzw. Methacrylsäure), empfiehlt sich die Suspensionskristallisation (insbesondere aus der Schmelze) als Reinigungsverfahren für die gewünschte Substanz, die ein sehr wirksames und kostengünstiges Verfahren bildet, um eine hohe Reinheit einer gewünschten chemischen Verbindung zu erzielen.

Dabei macht man sich zunutze, dass beim Wachstum der Kristalle in einer Flüssigkeit Verunreinigungen weitgehend aus dem Kristallgitter verdrängt werden und in der Mutterlauge zurückbleiben. Die Suspensionskristallisation weist dabei gegenüber der Schichtkristallisation den Vorteil auf, dass sie in einem kontinuierlichen Prozess durchgeführt werden kann. Außerdem ist die Reinheit der Kristalle aufgrund ihrer vergleichsweise langsamen Wachstumsgeschwindigkeit sehr hoch. Trotz der langsameren Wachstumsgeschwindigkeit kann mit der Suspensionskristallisation ein hoher Produktdurchsatz erzielt werden, da die Kristallisation aufgrund der großen Anzahl gleichzeitig wachsender Kristallite mit einer großen für das Wachstum zur Verfügung stehenden Gesamtfläche verbunden ist.

Bereits in einem einstufigen Kristallisationsprozess erhält man so hochreine Kristalle der gewünschten Verbindung. Prinzipiell kann die Suspensionskristallisation sowohl aus einer Lösung als auch aus einer Schmelze heraus erfolgen.

Ein entscheidender Schritt, der die Reinheit des kristallisierten Zielproduktes maßgeblich beeinflusst, ist dabei die Abtrennung der hochreinen Kristalle von ihrer Mutterlauge, die die Verunreinigungen in angereicherter Form und die nicht kristallisierten Anteile des Zielproduktes enthält, durch einen Fest/Flüssig-Trennprozess.

Bewährt hat sich für vorstehende Trennaufgabe die Verwendung von Waschkolonnen. Sie umfassen eine, in der Regel zylindrische, Wand (Einhüllende), die einen Prozessraum begrenzt.

Dem Prozessraum vorgelagert ist häufig ein Verteilerraum, in den die in der Waschkolonne aufzutrennende Kristallsuspension zugeführt wird. Auf ihrem Weg vom Verteilerraum in den Prozessraum wird die Kristallsuspension weitgehend gleichmäßig über den Querschnitt des Prozessraums verteilt. Im Prozessraum wird insbesondere durch Mutterlaugeentzug ein dichteres Kristallbett erzeugt und dieses durch den Prozessraum gefördert (dies kann bei Kolonnen mit erzwungenem Transport des Kristallbetts sowohl von oben nach unten als auch von unten nach oben erfolgen). Der Querschnitt des Prozessraums ist über seine Länge normalerweise konstant. Als Waschflüssigkeit wird eine Schmelze von zuvor in der Kolonne abgetrennten Kristallen (oder eine gesättigte Lösung derselben in Lösungsmitteln bzw. Lösungsmittelgemischen) im Gegenstrom durch das Kristallbett geleitet.

Zur Ausbildung eines dichten (kompakten) Kristallbetts in der Waschkolonne werden in der Praxis unterschiedliche Methoden angewendet. Bei gravitativ arbeitenden Waschkolonnen wird die Kristallsuspension von oben in die Kolonne eingeführt und das Kristallbett bildet sich in einem Sedimentationsprozess unter alleiniger Förderwirkung durch die Schwerkraft aus. Trennverfahren in derartigen Waschkolonnen sind nicht Gegenstand des erfindungsgemäßen Verfahrens.

Von solchen gravimetrischen Waschkolonnen unterscheiden sich Waschkolonnen mit erzwungenem Transport (bzw. Förderung) des Kristallbetts dadurch, dass in die Förderrichtung (bzw. Transportrichtung) des Kristallbetts wenigstens eine von der Gravitation verschiedene fördernd wirkende Kraft wirkt.

Grundsätzlich unterscheidet man Waschkolonnen mit erzwungenem Transport des Kristallbetts in Druckkolonnen (auch hydraulische Waschkolonnen oder Hydraulikkolonnen genannt) und in mechanische Kolonnen.

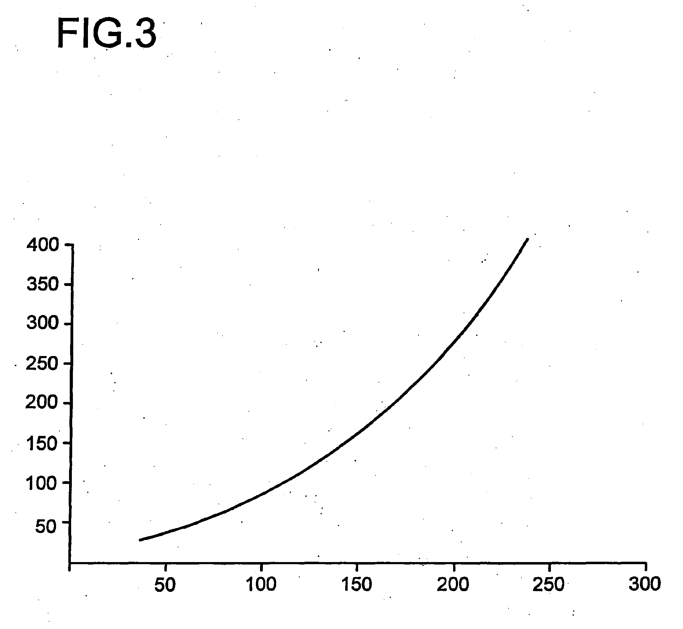

Mechanische Waschkolonnen enthalten eine mechanische Zwangsfördereinrichtung für die Kristalle. Dies kann im einfachsten Fall ein semipermeabler Stempel sein, der für die Mutterlauge durchlässig aber für die Kristalle in der zugeführten Suspension undurchlässig ist (vgl. Fig. 3 der WO 03/041832) und durch dessen Verschiebung der Druck zur Verdichtung und Förderung des Kristallbetts erzeugt wird. Die Verdichtung zu einem Kristallbett und dessen Förderung kann bei einer mechanischen Waschkolonne aber auch durch Abtrennung der Mutterlauge über Filter und mechanischen Transport der Kristalle vom Filter zum Kristallbett durch ein rotierendes Förderelement (z.B. Schnecken, Rührer, Wendeln oder Spiralen) erfolgen (vgl. Fig. 4 der WO 03/041832). Die Filter können dabei auch in die rotierenden Förderelemente integriert sein. Trennverfahren in mechanischen Waschkolonnen sind nicht Gegenstand des erfindungsgemäßen Verfahrens.

Bei den für das erfindungsgemäße Verfahren relevanten hydraulischen Waschkolonnen wird die Kristallsuspension am einen Ende der Waschkolonne in die unter Druck stehende zylinderförmige Waschkolonne gefördert (z.B. durch Pumpen (6) gemäß Fig. 1, oder durch hydrostatische Höhe). Parallel zur Zylinderachse erstreckt sich wenigstens ein Filterrohr ((2) in Fig. 1) durch die Kolonne, das von der Kristallsuspensionszufuhr wegweisend wenigstens ein Filter ((3) in Fig. 1) aufweist, das die einzige direkte Verbindung zwischen dem unter einem Druck P1 stehenden Filterrohrinneren und dem Inneren der Kolonne bildet.

Üblicherweise ragen die Filterrohre in die Waschzone hinein, sind in diesem Bereich der Waschkolonne jedoch nicht mehr hohl (vgl. z.B. WO 01/77056, WO 03/41833 und WO 03/41832). Man bezeichnet diesen Teil der Filterrohre auch als Filterrohrverdränger.

Die durch den Zuführkolonnendruck P2 > P1 aufgeprägte Flüssigkeitsströmung erzeugt eine Kompaktierung der Kristalle zu einem Kristallbett, sowie dessen Förderung.

Die Mutterlauge strömt über die Filter aus der Waschkolonne ab (jenseits der Filter kann Normaldruck, Unterdruck oder überatmosphärischer Druck herrschen). Das Kristallbett weist eine sogenannte Aufbaufront auf ((11) in Fig. 1), an der sich kontinuierlich Kristalle der eingeleiteten Kristallsuspension anlagern. Die Aufbaufront bezeichnet also den Übergang von der Suspension zum Kristallbett und ist durch einen relativ abrupten Anstieg des Kristallgehalts in der Suspension gekennzeichnet. Sie trennt die Suspensionszone ((17) in Fig. 1) vom Kristallbett. Vielfach wird die Aufbaufront auch als Filtrationsfront bezeichnet.

An dem der Aufbaufront gegenüberliegenden Ende des Kristallbetts wird kontinuierlich gewaschenes Kristallisat abgetragen ((14) in Fig. 1). Dies kann z.B. mittels einer Art Rotormesser oder mit Hilfe von Schabern erfolgen, die kontinuierlich Kristalle vom Kristallbett abtragen. Der kontinuierliche Kristallabtrag kann aber auch wie in der WO 03/063997 beschrieben erfolgen. Dort wird durch geeigneten Impuls der Waschschmelze eine kontinuierliche Deintegration von Kristallen aus dem Kristallbett bewirkt. Durch die kontinuierliche Anlagerung von Kristallen an der Aufbaufront einerseits und das kontinuierliche Abtragen von gewaschenen Kristallen an dem der Aufbaufront gegenüberliegenden Ende des Kristallbetts andererseits, wird die Transportrichtung des Kristallbetts definiert (sie kann sowohl von oben nach unten als auch von unten nach oben weisen). Die vom Kristallbett abgetragenen Kristalle werden, vorzugsweise nach ihrer Resuspendierung in Reinschmelze (bzw. in Lösungsmittel oder Lösungsmittelgemischen), durch Wärmeübertragung aufgeschmolzen (bzw. gelöst, vorzugsweise sättigend). Ein Teil der Schmelze (der Reinschmelze) wird als Reinproduktstrom abgeführt ((20) in Fig. 1) und ein anderer Teil der Reinschmelze wird als Waschflüssigkeit gegen die Transportrichtung des Kristallbetts an dessen der Aufbaufront abgewandten Ende in den Prozessraum rückgeführt (zurückgedrückt). Üblicherweise weist die Waschflüssigkeit dabei Schmelzpunkttemperatur auf. Vorzugsweise erfolgt die Resuspendierung in einem an das Kristallbettende angrenzenden (üblicherweise nicht mehr der Waschkolonne zugerechneten) separaten Raum (dem Suspendierraum), in den z.B. das Rotormesser die abgetragenen Kristalle einträgt ((21) in Fig. 1). Diese Suspension wird dann in zweckmäßiger Weise in einem Schmelzkreislauf ((22) in Fig. 1) über einen Wärmeträger ((15) in Fig. 1) geführt, über den auf indirektem Weg die zum Schmelzen der Kristalle erforderliche Wärme eingetragen wird. Häufig 70 bis 80 Gew.-%, in günstigen Fällen (z.B. bei ausgeprägter Rekristallisation in der Waschzone) sogar > 80 bis 100 Gew.% der geschmolzenen Kristalle werden als Reinprodukt ((20) in Fig. 1) aus dem Schmelzkreislauf abgeführt. Die Einstellung der entnommenen Menge an Reinprodukt erfolgt zweckmäßig über ein Produktregelventil ((23) in Fig. 1). Die Förderung im Schmelzkreis erfolgt vorteilhaft mit einer Förderpumpe ((24) in Fig. 1). Die Umlauf menge im Schmelzkreis beträgt vorteilhaft 2 bis 30, meist 5 bis 20 m³/h pro m³ (geschmolzen gerechnet) abgetragenem gereinigtem Kristallisat. D.h., die Resuspension weist anwendungstechnisch zweckmäßig einen niederen Kristallisatgehalt auf, was ihre Förderung begünstigt.

Wieviel Reinschmelze aus dem Schmelzkreis als Waschschmelze in die Waschkolonne dringt, wird zweckmäßig über den Druck im Schmelzkreis (Suspendierraum) geregelt (dieser wird indirekt über die Einstellung des Produktregelventils bestimmt). Im Regelfall ist es eine Teilmenge der abgetragenen Kristallisatmenge.

Das Aufschmelzen der abzutragenden Kristalle kann aber auch unmittelbar in der Waschkolonne vorgenommen werden (z.B. über entsprechend eingebaute Vorrichtungen zum Erwärmen am der Aufbaufront abgewandten Ende des Prozessraums). Es wird der Kolonne dann nur ein Teil der erzeugten Schmelze entnommen. Der andere Teil steigt in der hydraulischen Waschkolonne als Waschschmelze auf.

Durch die Förderung der Reinschmelze entgegengesetzt zur Förderrichtung des Kristallbetts wird das insbesondere mit der Mutterlauge getränkte Kristallbett praktisch in die Reinschmelze hineingedrückt (und umgekehrt) und die Mutterlauge im Kristallbett durch die Reinschmelze ("unter Ausbildung einer weitgehend stabilen Phasengrenze zwischen Reinschmelze und Mutterlauge") im wesentlichen einfach zurückgedrängt.

Im stationären Zustand (Betrieb) stellt sich als Ergebnis dieses Prozesses auf einer definierten Höhe des Kristallbetts (zwischen Filter und Kristallabtrag gelegen) eine Waschfront ("Phasengrenze") ein, die als derjenige Ort im Kristallbett definiert ist, wo die höchsten Temperatur und Konzentrationsgradienten auftreten (innerhalb der Waschfront erfolgt quasi der Phasenübergang von Reinschmelze nach Mutterlauge (bzw. Gemisch aus Mutter- und Steuerlauge); in der Waschfront springt die Temperatur praktisch von der tieferen Mutterlaugetemperatur auf die höhere Reinschmelzetemperatur; oberhalb und unterhalb der Waschfront liegen im wesentlichen konstante Temperaturen vor). Da in der Waschfront Reinschmelze und Mutterlauge (wie bereits gesagt) grob ausgedrückt aufeinanderprallen, erfolgt auf der Höhe der Waschfront auch ein Konzentrationssprung der unerwünschten Verunreinigungen von Mutterlaugenkonzentration zu Reinschmelzekonzentration. Der Bereich von der Waschfront bis zur Aufbaufront wird als Mutterlaugezone bezeichnet und der Bereich von der Waschfront bis zum der Aufbaufront abgewandten Ende des Kristallbetts wird als Reinschmelzezone bezeichnet.

Da die Kristallisationstemperatur in der verunreinigten Suspension unterhalb des Reinproduktschmelzpunktes liegt, kommt es bereits im Bereich der Waschfront außerdem zu einem Temperaturausgleich der kalten Kristalle mit der Waschschmelze, bei dem die Waschschmelze teilweise oder vollständig rekristallisiert. Dadurch wird wenigstens ein Teil der Waschschmelze zurückgewonnen. Der andere Teil verlässt die Waschkolonne gemeinsam mit der abgetrennten Mutterlauge durch die Filter und kann z.B. in die Gewinnung des zu reinigenden flüssigen Verbindungsgemisches rückgeführt, oder weitergereinigt ((25) in Fig. 1) und/oder gegebenenfalls wenigstens teilweise als im weiteren noch zu beschreibende Steuerlauge ((9) in Fig. 1) verwendet werden.

Die beschriebene Rekristallisation der Waschschmelze ist um so ausgeprägter, je weiter die Kristallisationstemperatur in der Mutterlauge unterhalb der Schmelztemperatur des Reinprodukts (bzw. der Sättigungstemperatur der Waschlösung) liegt (typische Temperaturunterschiede liegen bei 5 bis 15 K). Erfolgt quantitative Rekristallisation, können aus dem bereits beschriebenen Schmelzkreislauf ((22) in Fig. 1) 100% der geschmolzenen Kristalle als Reinprodukt abgeführt werden.

Der Querschnitt des Prozessraums einer hydraulischen Waschkolonne kann kreisförmig, oval oder eckig (z.B. regelmäßig vieleckig) sein.

Zur Erzielung einer adäquaten Reinigungswirkung in der Waschzone (der Teil des Kristallbetts der sich beginnend am Kristallabtrag bis zum Beginn des Filters erstreckt) muss die Waschfront in einer bestimmten Mindesthöhe oberhalb des Kristallabtrags positioniert sein. In ähnlicher Weise muss auch die Aufbaufront wohlpositioniert sein, um einen effektiven Betrieb einer hydraulischen Waschkolonne zu gewährleisten.

Da das Kristallbett durch die aus dem hydraulischen Strömungsdruckverlust der Mutterlauge (bzw. Mutter- und Steuerlauge) resultierende Kraft an den Filtern vorbei in die Waschzone jenseits der Filter transportiert wird, ist es für den Betrieb einer hydraulischen Waschkolonne energetisch ungünstig, wenn die Konzentrierungszone (der Bereich des Kristallbetts von der Aufbaufront bis zum Beginn der Waschzone) zu ausgedehnt ist. Mit zunehmender Länge der Konzentrierungszone wächst die Reibung und damit einhergehend der Strömungsdruckverlust. Umgekehrt ist eine unzureichende Länge der Konzentrierungszone insofern von Nachteil, als sie zur Ausbildung eines genügend kompaktierten Kristallbetts gegebenenfalls nicht ausreicht. Außerdem vermag ein zu geringer Strömungsdruckverlust das Kristallbett nicht in voll befriedigender Weise zu transportieren.

Zur Gewährleistung eines stabilen Betriebs einer hydraulischen Waschkolonne, d.h., zur Gewährleistung einer definierten Raum-Zeit-Ausbeute bei konstant guter Reinigungswirkung, ist eine stetige Kompensation äußerer Störgrößen erforderlich, die sich auf die Position von Wasch- und Aufbaufront auswirken. Derartige Störgrößen können beispielsweise Schwankungen des Mengenstroms der Suspension, Änderungen des Kristallgehalts in der Suspension, Variationen der Kristallgrößenverteilung oder auch Schwankungen in dem dem Kristallisator zugeführten Produktgemisch aus dem Syntheseprozess sein.

Die Position der Waschfront wird dabei üblicherweise durch die Einstellung der Waschschmelzemenge geregelt. Dies kann z.B. wie in den Schriften DE-A 100 36 881 und WO 02/09839 beschrieben erfolgen.

Da die Lage der Aufbau- bzw. Filtrationsfront durch die hydraulischen Verhältnisse (sie bestimmen die Vorschubgeschwindigkeit des Kristallbettes) in der Waschkolonne beeinflusst wird, bietet es sich gemäß den Lehren der DE-A 100 36 881 und WO 02/09839 an, einen Teil der über die Filter abgezogenen Mutterlauge (und gegebenenfalls Waschschmelze sowie gegebenenfalls Steuerlauge) zur Beeinflussung des hydraulischen Druckverlusts und damit zur Beeinflussung der Vorschubkraft in der Waschkolonne als Steuerlauge ((9) in Fig. 1) an dem zum Kristallabtrag entgegengesetzten Ende der Waschkolonne und/oder zwischen diesem Ende und dem Filterbeginn in diese zurückzupumpen (gegebenenfalls wird an mehreren Stellen gleichzeitig Steuerlauge zugepumpt). Der zurückzuführende Mengenstrom an Steuerlauge wird dabei zweckmäßig mittels einer entsprechenden Steuerstrompumpe ((8) in Fig. 1), z.B. durch Drehzahländerung und/oder ein zusätzliches Regelventil, eingestellt. Prinzipiell kommen als Steuerlauge aber auch von Mutterlauge verschiedene oder mit Mutterlauge identische, die zu reinigende Substanz enthaltende, Schmelzen und/oder Lösungen (die vorzugsweise mit der zu reinigenden Substanz gesättigt sind) in Betracht, die aus externen (d.h., nicht aus der Waschkolonne selbst bezogenen) Quellen (z.B. aus dem Kristallisator und/oder anderen Prozessstufen im Rahmen der Herstellung der zu reinigenden Substanz) bezogen werden können. Selbstverständlich können als Steuerlauge auch Gemische aus vorstehend und nachstehend angesprochenen Steuerlaugetypen eingesetzt werden. Anstelle mittels einer Pumpe kann die Steuerlauge auch durch hydrostatische Höhe in die Waschkolonne gedrückt werden. Natürlich können beide Zufuhrvarianten auch kombiniert angewendet werden. Beispielsweise kann als Steuerlauge auch Mutterlauge verwendet werden, die man unmittelbar aus dem zur Herstellung des Suspensionsstroms ((4) in Fig. 1) verwendeten Kristallisator bzw. der darin befindlichen Suspension entnimmt. Vorzugsweise weist die Steuerlauge keine höhere Reinheit auf als die Mutterlauge, um Rekristallisation in der Konzentrierungszone weitgehend zu vermeiden. D.h., auch die zur Suspensionskristallisation eingesetzte Ausgangsschmelze selbst kommt als Steuerlauge in Betracht. Ferner.kommen als Steuerlauge Flüssigkeiten in Betracht, in denen die zu reinigende Substanz einerseits schlecht bis gar nicht löslich ist, und die andererseits einen Festpunkt aufweisen, der unterhalb der Temperatur der der Waschkolonne zugeführten Kristallsuspension liegt. Steigt z.B. beim kontinuierlichen Betrieb das Kristallbett in der hydraulischen Waschkolonne gemäß Figur 1 an (verschiebt sich die Aufbaufront nach oben), wird der Mengenstrom der Steuerlauge (und mit diesem der hydraulische Strömungsdruckverlust der Mutter- und Steuerstromlauge in der Waschkolonne) erhöht und bei absinkendem Kristallbett wird er reduziert. Die Änderung des Mengenstroms der Steuerlauge kann dabei nach einer definierten Charakteristik durchgeführt werden, z.B. als eine lineare Mengenstromänderung mit der Zeit. Alternativ oder zusätzlich kann zur Regelung der Lage (der Position) der Aufbaufront in entsprechender Weise der Mengenstrom der Suspension (4) selbst erhöht oder reduziert werden.

Wird zwischen dem zum Kristallabtrag entgegensetzten Ende der Waschkolonne und dem Filterbeginn an mehreren Stellen (auf mehreren Höhen) Steuerlauge in die Waschkolonne gedrückt (dies muss dann normalerweise mit unterschiedlichen Drucken erfolgen; zweckmäßig können diese über Ventile eingestellt werden und entsprechen dem an der jeweiligen Zuführstelle in der Waschkolonne bestehenden Druck, bzw. liegen geringfügig oberhalb desselben) (dabei können die in die Waschkolonne gedrückten Steuerlaugen auch eine voneinander verschiedene chemische Zusammensetzung aufweisen; bevorzugt sind sie chemisch identisch) kann eine Erhöhung bzw. Verringerung des (Gesamt) Steuermengenstroms so ausgeführt werden, dass jeder einzelne der Steuerlaugenströme in seinem Betrag erhöht bzw. verringert wird. Mit Vorteil erfolgt vorgenannte Erhöhung bzw. Verringerung dabei so, dass das Verhältnis der an den unterschiedlichen Stellen pro Zeiteinheit zugeführten Steuerstromvolumina untereinander konstant bleibt. Selbstredend können die Steuerlaugenströme auch "Blindströme" umfassen. Dies sind Steuerlaugenströme, deren Betrag unabhängig vom Verhalten der Aufbaufront stabil gehalten wird. Sie bilden quasi einen Grundbeitrag zur Steuerung. Vorzugsweise wird man nur einen Steuerstrom verwenden, und diesen vorzugweise am zum Kristallabtrag entgegengesetzten Ende der Waschkolonne in diese (in die Suspensionszone) drücken (pumpen) (mit dem Druck P 2). Mit Vorteil besteht dieser Steuerstrom ausschließlich aus über die Filterrohre aus der Waschkolonne herausgeführter Lauge ((9) in Fig. 1) und wird zweckmäßig über die Steuerpumpe S ((8) in Fig. 1) in die Waschkolonne geführt (gepumpt).

Prinzipiell kann der Steuerlaugestrom aber auch dadurch in die Waschkolonne geführt werden, dass man ihn (z.B. vor und/oder nach der Pumpe (6)) dem der Waschkolonne zuzuführenden Suspensionsstrom (4) zuführt.

Zur Überwachung der Position der Aufbau- bzw. Filtrationsfront empfehlen sowohl die WO 02/09839 als auch die DE-A 100 36 881 eine optische Positionsdetektierung. Nachteilig an einer optischen Positionsdetektierung ist jedoch, dass wenigstens dort, wo sie zu erfolgen hat, die zylindrische Einhüllende der Waschkolonne aus einem transparenten Material (z.B. Glas) bestehen muss. Bevorzugt (vgl. z.B. WO 03/041832) ist die den Prozessraum einer hydraulischen Waschkolonne Einhüllende jedoch aus Metall gefertigt. Zwar können in einer Metallwand prinzipiell transparente Fenster eingebaut werden, doch ist eine druckdichte (wie es im Fall einer hydraulischen Waschkolonne erforderlich ist) Fertigung derselben fertigungstechnisch nicht einfach und ihre Anwendung insbesondere bei Gefahrstoffen sicherheitstechnisch nicht unbedenklich. Darüber hinaus ist eine punktuelle Überwachung durch Fenster mit begrenzter Sicht hindurch nicht voll befriedigend. Erstrebenswert ist vielmehr eine Überwachungsvariante, die die gesamte Länge der Konzentrierungszone erfasst (einblickt). Zusätzlich sind optische Sensoren vergleichsweise teuer.

Die Aufgabe der vorliegenden Erfindung bestand daher darin, ein verbessertes Verfahren zur Regelung der Position der Aufbaufront in einer hydraulischen Waschkolonne zur Verfügung zu stellen, bei dem zur Festlegung des zum jeweiligen Zeitpunkt in die hydraulische Waschkolonne zu führenden Mengenstroms an Steuerlauge und/oder Suspension eine vorteilhaftere Messgröße als im Stand der Technik verwendet wird.

Demgemäss wurde ein Verfahren zur Regelung einer hydraulischen Waschkolonne, die eine zylindrische Einhüllende (1) aufweist, die die Kolonne begrenzt und in der sich parallel zur Zylinderachse ein oder mehrere Filterrohre (2) durch die Kolonne erstrecken, die in der Nähe des zweiten Endes der Kolonne in der Filterrohrwand wenigstens ein Filter (3) aufweisen, das die einzige direkte Verbindung zwischen dem unter Druck P1 stehenden Filterrohrinneren und dem Inneren der Kolonne bildet, bei dem man
- wenigstens einen Strom einer Suspension (4), die in einer Mutterlauge suspendierte Kristalle einer zu reinigenden Substanz enthält, am ersten Ende der Kolonne (5) mit einem Druck P2 (z.B. mittels einer Pumpe (6)), der größer als P1 ist, in selbige kontinuierlich einspeist,
- über die Filter Mutterlauge (7) in das Filterrohrinnere hinein und über die Filterrohre aus der Kolonne herausführt,
- am ersten Ende der Kolonne und/oder zwischen diesem Ende und dem Filterbeginn gegebenenfalls Steuerlauge (9) in die Waschkolonne führt,
- durch die Mutter- und gegebenenfalls Steuerlaugeführung in der Kolonne ein Kristallbett (10) der zu reinigenden Substanz ausbildet, das eine dem ersten Ende der Kolonne zugewandte Aufbaufront (11) aufweist, an der sich kontinuierlich Kristalle der eingeleiteten Suspension an das Kristallbett anlagern,
- das Kristallbett durch die aus dem hydraulischen Strömungsdruckverlust der Mutter- und gegebenenfalls Steuerlaugeführung in der Kolonne resultierende Kraft an den Filtern vorbei in die zwischen den Filtern und dem zweiten Ende der Waschkolonne (12) befindliche Waschzone transportiert (13),
- an dem der Aufbaufront gegenüberliegenden Ende des Kristallbetts kontinuierlich Kristalle abträgt (14),
- die abgetragenen Kristalle aufschmilzt (15) und einen Teil der Schmelze als Waschflüssigkeitsstrom vom zweiten Ende der Kolonne herkommend gegen die Transportrichtung der Kristalle durch das Kristallbett leitet (16) und
- die Position der Aufbaufront mit Hilfe des Mengenstroms von in die Waschkolonne geführter Steuerlauge und/oder mit Hilfe des Mengenstroms der in die Waschkolonne geführten Suspension (4) regelt, gefunden,
das dadurch gekennzeichnet ist,
dass man zur Festlegung (Regelung) der Mengenströme der in die Waschkolonne geführten Steuerlauge und/oder Suspension (4) wenigstens einen Druckunterschied ΔP_{SK} verwendet (heranzieht), der in der Kolonne zwischen wenigstens einem Punkt in der vor der Aufbaufront befindlichen Suspensionszone (17) und wenigstens einem Punkt in der von der Aufbaufront bis zum Ende des Kristallbetts ragenden Zone (vorzugsweise in der bis zum Beginn der Waschzone (19) ragenden Konzentrierungszone (18) des Kristallbetts) besteht.

Erfindungsgemäß bevorzugt werden die Druckunterschiede ΔP_{SK} in ihrer um den hydrostatischen Druckunterschied bereinigten Form ΔP_{SKB} zur Festlegung des Mengenstroms der in die Waschkolonne geführten Steuerlauge und/oder Suspension (4) herangezogen.

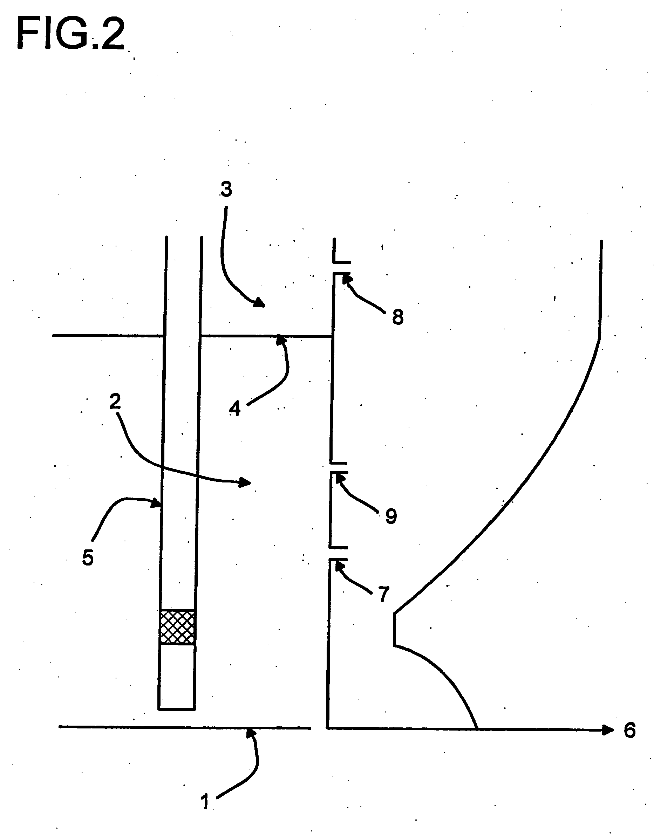

Grundlage für das erfindungsgemäße Verfahren sind die in Fig. 2 für eine hydraulische Waschkolonne schematisch dargestellten, um den hydrostatischen Druck bereinigten, Druckverhältnisse. Dabei ist die Bedeutung der numerischen Adressen wie folgt
- 1 =: Mittel (z.B. Rotormesser) zum kontinuierlichen Abtragen von Kristallen vom Kristallbett
- **2**: = Kristallbett
- 3 =: Suspensionszone
- 4 =: Aufbaufront
- 5 =: Filterrohr
- 6 =: um den hydrostatischen Druck bereinigter Druck innerhalb der hydraulischen Waschkolonne in Abhängigkeit von deren Länge

Der Wortlaut "um den hydrostatischen Druck bereinigter Druck" meint dabei den an einem bestimmten Punkt der hydraulischen Waschkolonne herrschenden Druck abzüglich des in diesem Punkt herrschenden "hydrostatischen" Druckes. Sprachlich vereinfacht soll dieser Druck in dieser Schrift als "bereinigter Druck" P bezeichnet werden. Der Begriff hydrostatischer Druck meint dabei den durch das Gewicht der über diesem Punkt befindlichen Säule ausgeübten Druck.

Innerhalb der Suspensionszone ist der bereinigte Druck im wesentlichen konstant und befindet sich auf Treibdruckniveau P2. Anschließend fällt er hinter der Aufbaufront innerhalb des Kristallbetts (innerhalb der Konzentrierungszone) bis auf Filterhöhe progressiv ab.

Auf Höhe des Kristallabtrags weist der bereinigte Druck Waschdruckniveau auf. Das ist der Druck, mit dem die Waschschmelze in die Waschkolonne rückgeführt wird. Dieser Druck fällt innerhalb der Waschzone gleichfalls ab (häufig annähernd linear). Treibdruck und Waschdruck stehen in einem von der Reibung abhängigen festen Verhältnis zueinander.

Bestimmt man nun die Differenz zwischen dem bereinigten Druck eines Punktes innerhalb der Suspensionszone und dem bereinigten Druck eines Punktes innerhalb der von der Aufbaufront bis zum Ende des Kristallbetts ragenden Zone (vorzugsweise innerhalb der Konzentrationszone), so ist dieser Druckunterschied ΔP_{SKB} ein Maß für den momentanen Betriebszustand der Waschkolonne und kann in vorteilhafter Weise zur Festlegung der in die Waschkolonne zu führenden Mengenströme an Steuerlauge und/oder Suspension herangezogen werden.

Bei großen Werten ΔP_{SKB}, wie sie in der Praxis aus Gründen einer möglichst hohen Raum-Zeit-Ausbeute und daraus resultierend möglichst hohen Kolonnendurchsätzen bzw. Treibdrucken häufig vorliegen, ist der aus der hydrostatischen Druckdifferenz resultierende Anteil am zu einem ΔP_{SKB}-Wert gehörigen unbereinigten ΔP_{SK}-Wert klein und in der erforderlichen bzw. angestrebten Regelschärfe vernachlässigbar.

In diesen Fällen können für das erfindungsgemäße Verfahren auch die unmittelbaren Werte ΔP_{SK} in ihrer um die hydrostatische Druckdifferenz (um den hydrostatischen Differenzdruck) unbereinigten Form zur Festlegung der Mengenströme der in die Waschkolonne zu führenden Steuerlauge und/oder Suspension (4) herangezogen werden.

Erfindungsgemäß bevorzugt werden jedoch, insbesondere im Sinne einer Regelung der der Waschkolonne zuzuführenden Mengenströme an Steuerlauge und/oder Suspension (4) mit erhöhter Präzision, um den hydrostatischen Differenzdruck bereinigte Werte ΔP_{SK}, d.h., Werte ΔP_{SKB}, für diese Festlegung herangezogen.

In den nachfolgenden Ausführungen steht daher ΔP_{SK} stellvertretend für "ΔP_{SK}" und "ΔP_{SKB}", soweit nichts anderes gesagt wird. D.h., alles was im folgenden als für ΔP_{SK} gültig ausgeführt wird, gilt so auch für das zugehörige ΔP_{SKB}.

Erfindungsgemäß bevorzugt befinden sich alle zur Bestimmung eines ΔP_{SK} herangezogenen, in der von der Aufbaufront bis zum Ende des Kristallbetts ragenden Zone liegenden, Bezugspunkte im Bereich zwischen dem Kristallabtrag und der dem Kristallabtrag nächstliegenden Zufuhrstelle eines Steuerstroms, der keinen Blindstrom bildet. Erfindungsgemäß zweckmäßig ist diese nächstliegende Zufuhrstelle um wenigstens ein Fünftel, vorzugsweise um wenigstens ein Viertel, und besonders bevorzugt um wenigstens ein Drittel der Länge der Konzentrierungszone vom Filterbeginn entfernt (von der Aufbaufront her betrachtet).

Verschiebt sich nun die Lage der Aufbaufront innerhalb der Waschkolonne aufgrund einer Störung, so ändert sich der Wert für ΔP_{SK} (vorausgesetzt die Position der beiden Messpunkte innerhalb der Waschkolonne wird beibehalten). Beginnt die Aufbaufront in Figur 2 nach unten zu wandern, wird ΔP_{SK} kleiner. Dies ist das Signal, den Steuerlaugemengenstrom zu verringern und/oder den Suspensionsstrom (4) zu erhöhen. Beginnt die Aufbaufront in Figur 2 nach oben zu wandern, wird ΔP_{SK} größer. Dies ist das Signal, den Steuerlaugemengenstrom zu erhöhen und/oder den Suspensionsstrom (4) zu verringern.

Experimentell ist ΔP_{SK} in einfacher Weise dadurch zugänglich, dass man in die zylindrische Einhüllende zwei offene (am Umfang der Waschkolonne vorzugsweise übereinander, d.h. gleich positionierte) Bohrungen anbringt. Die eine der beiden Bohrungen befindet sich auf einer Höhe der Suspensionszone (z.B. (8) in Fig. 2) und die andere der beiden Bohrungen befindet sich z.B. auf einer Höhe der Konzentrierungszone (z.B. (7) in Fig. 2) bzw. allgemein auf einer Höhe innerhalb der von der Aufbaufront bis zum Ende des Kristallbetts ragenden Zone. Sich an die offenen Bohrungen anschließende Leitungen führen jeweils zum Messkopf, der unmittelbar die zwischen beiden Bohrstellen herrschende Druckdifferenz feststellt und diese in der Regel in ein anderes Signal, z.B. in ein elektrisches Signal, umformt.

Werden die beiden Leitungen am Messkopf auf ein und dieselbe Höhe geführt, enthält die im Messkopf detektierte Druckdifferenz den zwischen den beiden Bohrungen bestehenden hydrostatischen Druckunterschied normalerweise nicht mehr, d.h., sie ist durch das angewandte Messverfahren automatisch um selbigen bereinigt. In diesem Fall liefert die Messung unmittelbar ΔP_{SKB}. Selbstverständlich kann auch jede Leitung zu einem ihr zugeordneten separaten Messkopf geführt werden. Befinden sich die Messköpfe auf gleicher Höhe, resultiert das relevante ΔP_{SKB} durch einfache Differenzbildung der in den Messköpfen festgestellten Drucke. In besonders einfacher Weise kann ΔP_{SKB} mit Hilfe einer Druckdifferenzmessmembran ermittelt werden. Eine der beiden Leitungen endet auf der linken Seite der Membran und die andere der beiden Leitungen endet auf der rechten Seite der Membran. Der die Membran enthaltende Messkopf zeigt unmittelbar ΔP_{SKB} an.

Vorzugsweise sind die beiden Leitungen mit Flüssigkeit gefüllt, damit sie nicht verstopfen. Als solche Flüssigkeiten kommen z.B. Mutterlauge und Reinschmelze in Betracht. Im Fall einer Suspension von wasserlöslichen Kristallen (z.B. von Acrylsäurekristallen) ist aber auch eine Verwendung von Wasser als solche Füllflüssigkeit zweckmäßig. Zum einen verfügt Wasser im Unterschied zu Acrylsäure über keine Neigung zur radikalischen Polymerisation und außerdem verfügt Wasser über ein ausgeprägtes Lösungsvermögen für an oder in den offenen Bohrungen anwachsende Acrylsäurekristalle. Vorzugsweise enthält das Wasser eine geringe Menge an Polymerisationsinhibitor gelöst (z.B. Phenothiazion, Monomethylether des Hydrochinons, p-Nitrosophenol, Hydrochinon, Nitrosodiethylanilin, 4-Hydroxy-2,2,6,6-tetramethylpiperidin-N-Oxid und/oder 4-Oxo-2,2,6,6-tetramethylpiperidin-N-Oxid). Zweckmäßig wird die wässrige Füllung der Leitungen während des kontinuierlichen Betriebs der Waschkolonne von Zeit zu Zeit oder kontinuierlich ausgetauscht. Mit Vorteil schließt die jeweilige Bohrung auf der Innenseite der Einhüllenden der hydraulischen Waschkolonne glatt ab, um keinen Widerstand beim Transport des Kristallbetts zu bilden.

Der Durchmesser der Eintrittsöffnung solcher offenen Bohrungen beträgt vom Kolonneninneren her gesehen zum Zweck der Messung von ΔP_{SK} anwendungstechnisch zweckmäßig ≤ 5mm, oft ≤ 3mm und in der Regel ≥ 0,1 mm. Durch die Wandung hindurch kann anwendungstechnisch zweckmäßig ein sich zum Kolonneninneren hin kontinuierlich oder stufenförmig verjüngender Bohrungsdurchmesser angewendet werden.

Erfindungsgemäß bevorzugt befindet sich eine längs der Konzentrierungszone angebrachte offene Bohrung, bezogen auf die Gesamtlänge derselben, wenigstens ein Längendrittel, besonders bevorzugt wenigstens zwei Längendrittel und ganz besonders bevorzugt wenigstens drei Längenviertel jenseits der Aufbaufront. Selbstverständlich kann sich die offene Bohrung längs der Konzentrierungszone auch auf Filterhöhe befinden: Eine Positionierung des nicht in der Suspensionszone befindlichen ΔP_{SK} Bezugspunktes innerhalb der Waschzone ist erfindungsgemäß weniger bevorzugt.

Natürlich kann der hydrostatische Druckunterschied auch rechnerisch (die Massendichten sind bekannt) ermittelt und vom gemessenen Druckunterschied ΔP_{SK} abgezogen werden (z.B. dann, wenn die Leitungen von den offenen Bohrungen zu auf unterschiedlichen Höhen befindlichen Druckmessköpfen geführt werden).

Selbstverständlich können auf ein- und derselben Höhe längs der Konzentrierungszone (bzw. allgemein längs der von der Aufbaufront bis zum Ende des Kristallbetts ragenden Zone), um den Umfang der hydraulischen Waschkolonne (vorzugsweise gleichmäßig) verteilt, auch mehrere offene Bohrungen angebracht werden. Auf diese Weise können simultan mehrere Druckunterschiede ΔP_{SK} gleichzeitig ermittelt und zur Festlegung (Regelung) des Steuerlaugemengenstroms und/oder Suspensionsstroms (4) herangezogen werden.

Erfindungsgemäß vorteilhaft verwendet man zur Festlegung des Mengenstoms der in die Waschkolonne geführten bzw. zurückgepumpten Steuerlauge und/oder zur Festlegung des Mengenstroms des in die Waschkolonne geführten Suspensionsstroms (4) das Verhältnis V zweier Druckunterschiede (ΔP_{SK})¹ und (ΔP_{SK})² (die vorzugsweise beide um den jeweils zugehörigen hydrostatischen Druckunterschied bereinigt sind), die dadurch ausgezeichnet sind, dass sich ihre jeweiligen Bezugspunkte in der Konzentrierungszone (bzw. allgemein in der von der Aufbaufront bis zum Ende des Kristallbetts ragenden Zone) auf unterschiedlicher Höhe befinden. Ihre jeweiligen Bezugspunkte in der Suspensionszone befinden sich bevorzugt auf gleicher Höhe. Prinzipiell können diese jedoch auch voneinander verschiedene Höhenlagen einnehmen. Auch kann für beide Druckunterschiede ein- und derselbe Bezugspunkt in der Suspensionszone verwendet werden.

Im stationären Betriebszustand wird dieses Verhältnis V einen Wert > 0 (der Wert 0 entspricht einer verschwindenden Kristallbettlänge) und < 1 (der Wert 1 entspricht einer unbegrenzten Kristallbettlänge), häufig ≥ 0,1 (bzw. ≥ 0,2) und ≤ 0,8 aufweisen. Dies gilt insbesondere dann, wenn die beiden nicht in der Suspensionszone liegenden Bezugspunkte in der Konzentrierungszone liegen.

Verändert sich die Lage der Aufbaufront, verändert sich der Wert von V und löst eine Veränderung des Mengenstroms der Steuerlauge und/oder Suspension (4) aus. Wander die Aufbaufront in Figur 1 nach oben, wächst V und löst eine Zunahme des zugeführten Mengenstroms an Steuerlauge und/oder eine Abnahme des zugeführten Mengenstroms der Suspension (4) aus. Wandert die Aufbaufront in Figur 1 nach unten, sinkt V und löst eine Abnahme des zugeführten Mengenstroms an Steuerlauge und/oder eine Zunahme des zugeführten Mengenstroms der Suspension (4) aus.

Der Vorteil einer Verwendung von V als Indikator für eine Lageveränderung der Auf baufront besteht darin, dass von einer Veränderung der Lage der Aufbaufront verschiedene Ursachen (z.B. eine veränderte durch das Kristallbett strömende Flüssigkeitsmenge und/oder geänderte Kristallformen und/oder -größen) für eine Veränderung von (ΔP_{SK} sich in gleicher Weise auf (ΔP_{SK})¹ und (ΔP_{SK})² auswirken und sich deshalb bei der Bildung des Quotienten V in ihrer Auswirkung auf diesen neutralisieren.

Die experimentelle Zugänglichkeit von (ΔP_{SK})¹ und (ΔP_{SK})² ist in einfacher Weise z.B. dadurch möglich, dass man in einer hydraulischen Waschkolonne gemäß Figur 2 längs der Konzentrierungszone (bzw. allgemein in der von der Aufbaufront bis zum Ende des Kristallbetts ragenden Zone) oberhalb der offenen Bohrung (7) eine weitere offene Bohrung (9) anbringt (vorzugsweise werden die offenen Bohrungen (7), (9) und (8) am Umfang der hydraulischen Waschkolonne nicht gegeneinander versetzt, sondern unmittelbar untereinander befindlich angebracht).

Von der offenen Bohrung (8) führt dann z.B. eine sich verzweigende Druckleitung mit je einem der beiden Zweige zur linken Seite je einer Druckmessmembran. Auf die rechte Seite der einen Druckmessmembran führt dann z.B: die Druckleitung von der offenen Bohrung (9) und auf die rechte Seite der anderen Druckmessmembran führt in entsprechender Weise die Druckleitung von der offenen Bohrung (7).

Selbstredend können um den Umfang der hydraulischen Waschkolonne mehrere, z.B. bis zu 5 oder 4 (vorzugsweise auf identische Höhen gebohrte) Tripel an offenen Bohrungen (7), (8) und (9) angebracht sein. Auf diese Weise ist simultan ein ganzer Satz von Verhältnissen V erhältlich. Die Mengenströme an Steuerlauge und/oder Suspension (4) wird man anwendungstechnisch zweckmäßig nur dann erhöhen oder verringern, wenn die Mehrzahl der Verhältnisse V eine messtechnisch auflösbare Abweichung in die gleiche Richtung aufweist.

Der Treibdruck (angegeben als Überdruck gegen Atmosphäre) in einer hydraulischen Waschkolonne beträgt häufig bis zu 10 bar, vielfach bis zu 6 bar und oft 1 bis 5 bar bzw. 0,5 bis 4 bar. Der hydraulische Strömungsdruckverlust der Mutterlauge beträgt in der Regel ≥ 100 mbar bis ≤ 5 bar bzw. ≤ 10 bar.

die Messerdrehzahl liegt meist bei Werten > 0 und ≤ 100/min, bzw. ≤ 60/min. Die Temperatur im Schmelzkreis liegt üblicherweise 0,01 bis 5°C, häufig 0,1 bis 3°C oberhalb der Schmelztemperatur der abgetragenen gewaschenen Kristalle.

Die Gesamthöhe des Kristallbetts in einer hydraulischen Waschkolonne beträgt typisch 300 bis 2000 mm, häufig 500 bis 1500 mm, vielfach 400 mm bis 1000 mm. Die Länge der Filterelemente beträgt häufig 20 bis 200 mm. Die Filterperforation betreffend kann den Ausführungen auf Seite 7 der WO 03/041833 gefolgt werden. Die Länge der Filter rohrverdränger beträgt regelmäßig 100 bis 500 mm. Typische Innendurchmesser einer hydraulischen Waschkolonne liegen bei 300 bis 3000 mm. Typische Innendurchmesser der Filterrohre betragen 5 bis 200 mm, häufig 10 bis 100 mm, vielfach 20 bis 80 mm.

Die Anzahl der Filterrohre kann im Fall einer großtechnischen Anwendung erfindungsgemäß 3 bis 200 oder mehr betragen. Die Länge der Waschzone liegt typisch beim 0,5- bis 20-fachen, bevorzugt beim 1- bis 8-fachen und ganz besonders bevorzugt beim 2- bis 5-fachen des Abstandes des der Einhüllenden nächstliegenden Filterrohres zur Einhüllenden (in der Regel beträgt dieser Abstand 25 bis 500 mm, häufig 40 bis 250 mm, oft 80 bis 200 mm).

Erfindungsgemäß ist es vorteilhaft, wenn beim Verhältnis V zweier (vorzugsweise bereinigter) Druckunterschiede (ΔP_{SK})¹ und (ΔP_{SK})² der Abstand der beiden vorzugsweise in der Konzentrierungszone (bzw. allgemein in der von der Aufbaufront bis zum Ende des Kristallbetts ragenden Zone) befindlichen Bezugspunkte (der Abstand der entsprechenden offenen Bohrungen) 10 bis 1000 mm, vorzugsweise 50 bis 500 mm und besonders bevorzugt 100 bis 300 mm bzw. 200 mm beträgt. Der Abstand des der Auf baufront nächstliegenden der beiden vorzugsweise in der Konzentrierungszone befindlichen Bezugspunkte zur Aufbaufront beträgt erfindungsgemäß zweckmäßig 100 bis 2000 mm, vorteilhaft 200 bis 1000 mm.

Die Vorzüge der erfindungsgemäßen Verfahrensweise liegen insbesondere in seiner Wirtschaftlichkeit und in der Tatsache, dass es sich um ein kontinuierlich anwendbares Regel- bzw. Steuerverfahren handelt. Es ist insbesondere in hydraulischen Waschkolonnen anwendbar, wie sie in der WO 03/041833 und in der WO 03/041832 beschrieben sind. Die Differenztemperatur zwischen Mutterlauge und Reinschmelze kann bis zu 15°C und mehr betragen. Vielfach beträgt die Differenztemperatur 4 bis 10°C und bei geringem Verunreinigungsgehalt der Mutterlauge oft auch nur 2 bis 4°C.

Das erfindungsgemäße Verfahren eignet sich, wie bereits erwähnt, insbesondere zum reinigenden Abtrennen von Acrylsäurekristallen (bzw. Methacrylsäurekristallen) aus ihrer Suspension in verunreinigten Acrylsäureschmelzen (bzw. Methacrylsäureschmelzen), wie sie in der WO 01/77056, WO 02/055469 und der WO 03/078378 beschrieben sind. Dies vor allem dann, wenn die Acrylsäure-Kristalle eine würfel- bis quaderförmige Erscheinungsform aufweisen, und dabei ein Länge (L) zu Dicke (D) Verhältnis im Bereich L:D = 1:1 bis L:D = 6:1, bevorzugt im Bereich 1:1 bis 4:1, und besonders bevorzugt im Bereich 1,5:1 bis 3,5:1 zeigen. Die Dicke D der Kristalle liegt dabei üblicherweise im Bereich von 20 bis 600 µm, oft 50 bis 300 µm. Die Länge L der Kristalle liegt üblicherweise im Bereich von 50 bis 1500 µm, oft bei 200 bis 800 µm.

Das sind insbesondere Suspensionen, die z.B. durch Suspensionskristallisation von Rohacrylsäuren erhältlich sind, die enthalten:
65, oder 70, oder 75, oder 85 bis 99,5 Gew.-% Acrylsäure,
≥ 0, in der Regel 0,1 bis 40 Gew.-%, oder bis 20 Gew.-% Wasser,
≥ 0, in der Regel 0,001 bis 5 Gew.-% Acrolein,
≥ 0, in der Regel 0,001 bis 10 Gew.-% Methacrolein,
≥ 0, in der Regel 0,001 bis 10 Gew-% Methacrylsäure,
≥ 0, in der Regel 0,01 bis 5 Gew.-% Essigsäure,
≥ 0, in der Regel 0,01 bis 5 Gew.-% Propionsäure,
≥ 0, in der Regel 0,001 bis 5 Gew.-% Formaldehyd,
≥ 0, in der Regel 0,001 bis 5 Gew.-% weitere Aldehyde (unter Umständen auch je Aldehyd, z.B. Benzaldehyd), und
≥ 0, in der Regel 0,01 bis 5 Gew.-% Maleinsäure.

Insbesondere sind es Suspensionen, die z.B. durch Suspensionskristallisation von Rohacrylsäuren erhältlich sind, die enthalten:
≥ 70 Gew.-% Acrylsäure,
bis zu 20 Gew.-% Wasser,
bis zu 15 Gew.-% Essigsäure,
bis zu 5 Gew.-% Propionsäure,
bis zu 5 Gew.-% niedermolekulare Aldehyde,
bis zu 3 Gew.-% Polymerisationsinhibitoren und
bis zu 5 Ges.-% Acrylsäure-Oligomere (Michael-Addukte).

Vor allem sind es Suspensionen, die z.B. durch Suspensionskristallisation von Rohacrylsäuren erhältlich sind, die enthalten:
90 bis 98 Gew.-% Acrylsäure,
0,2 bis 5 Gew.% Wasser,
0,001 bis 3 Gew.-% Acrolein,
0,001 bis 3 Gew.-% Methacrolein,
0,01 bis 3 Gew.-% Essigsäure,
0,001 bis 3 Gew.-% Propionsäure,
0,001 bis 3 Gew.-% weitere Aldehyde, und
0,001 bis 3 Gew.-% Maleinsäure.

Dabei ist es vorteilhaft, die Suspensionskristallisation in wenigstens zwei, oder in wenigstens drei parallel betriebenen Suspensionskristallisatoren durchzuführen. Aus diesen werden die Kristallsuspensionen in einen gemeinsamen Pufferbehälter geführt, in welchem die resultierende Gesamtkristallsuspension kontinuierlich homogenisiert, gemischt wird. Aus dem Pufferbehälter heraus werden dann vorteilhaft wenigstens zwei, besonders vorteilhaft wenigstens drei parallel betriebene hydraulische Waschkolonnen gespeist. Sowohl die Inbetriebnahme der parallel betriebenen Suspensionskristallisatoren, als auch die Inbetriebnahme der parallel betriebenen hydraulischen Waschkolonnen kann untereinander zeitversetzt oder parallel erfolgen. Verkrustungsbildungen erfolgen dann ebenfalls zeitversetzt und können in entsprechender Weise zeitversetzt beseitigt werden.

Die einzelne Waschkolonne und der einzelne Suspensionskristallisator wird dabei ingenieurmäßig so geplant, dass zwei in Betrieb befindliche auch die Mengen des Dritten, gegebenenfalls ausgefallenen, übernehmen können.

Bevorzugt zu verwendende Suspensionskristallisatoren sind z.B. in der WO 04/35514 beschrieben.

Das erfindungsgemäße Verfahren eignet sich aber auch im Fall anderer Kristallsuspensionen, wie sie z.B. in der EP-A 97405 mit Xylolkristallsuspensionen beschrieben sind. Günstig ist es auch im Fall von N-Vinylpyrrolidonkristallsuspensionen.

Ein wesentlicher Vorteil der erfindungsgemäßen Verfahrensweise ist, dass sie auch bei hydraulischen Waschkolonnen, die eine metallische Einhüllende aufweisen, angewendet werden kann. Dabei kann es sich sowohl um Reinmetalle, aber auch um Legierungen, z.B. Kohlenstoffstähle, Eisenbasislegierungen (Edelstahl, z.B. mit Cr/Ni-Zumischung) oder um Nickelbasislegierungen (z.B. Hastelloy Qualitäten) handeln. Handelt es sich bei der reinigend abzutrennenden Substanz um Acrylsäure, wird als Wandmaterial Edelstahl, insbesondere Edelstahl 1.4571, oder 1.4541, oder 1.4306, oder 1.4404 bevorzugt. Die Stärke der den Prozessraum begrenzenden Metallwand beträgt in zweckmäßiger Weise 3 bis 30 mm, häufig 4 bis 20 mm und meist 5 bis 15 mm. Letzteres gilt insbesondere im Fall von Edelstahl.

Das erfindungsgemäße Verfahren ist aber auch dann anwendbar, wenn die Einhüllende der hydraulischen Waschkolonne aus Glas oder aus synthetischem Kunststoff gefertigt ist.

Typische Werte ΔP_{SK} oder ΔP_{SKB} betragen beim erfindungsgemäßen Verfahren häufig 50 bis 8000 mbär, vielfach 100 bis 4000 mbar, oft 200 bis 1000 mbar, bzw. bis 750 mbar.

Typische Werte V betragen beim erfindungsgemäßen Verfahren 0,1 bis 0,8, häufig 0,2 bis 0,4.

Auf vorgenannte Werte kann beim erfindungsgemäßen Verfahren geregelt werden.

Selbstverständlich kann das erfindungsgemäße Regelverfahren gemeinsam mit den in der DE-A 10036881 und WO 02/09839 offenbarten Regelverfahren angewendet werden.

Als solches alternatives Verfahren kommt auch eine radiometrische Methode zur Festlegung der Mengenströme an Steuerlauge und/oder Suspension (4) in Betracht. Dabei wird ein y-Strahler eingesetzt, der in das Kristallbett hineinstrahlt. Die Extinktion der Strahlung beim Durchgang durch das Kristallbett ist von der Länge des Kristallbetts und damit von der Position der Aufbaufront abhängig. Damit können die Mengenströme an Steuerlauge und/oder Suspension (4) auch in einfachster Weise gemäß der Schwächung des Strahlungssignals geregelt (festgelegt) werden.

Erfindungsgemäß bevorzugt wählt man einen im wesentlichen stationären Suspensionsstrom (4) und regelt die Position der Aufbaufront im wesentlichen ausschließlich über den Betrag des Mengenstroms der Steuerlauge. D.h., das erfindungsgemäße Verfahren umfasst insbesondere Verfahren zur Regelung einer hydraulischen Waschkolonne, die eine zylindrische Einhüllende (1) aufweist, die die Kolonne begrenzt und in der sich parallel zur Zylinderachse ein oder mehrere Filterrohre (2) durch die Kolonne erstrecken, die in der Nähe des zweiten Endes der Kolonne in der Filterrohrwand wenigstens ein Filter (3) aufweisen, das die einzige direkte Verbindung zwischen dem unter Druck P1 stehenden Filterrohrinneren und dem Inneren der Kolonne bildet, bei dem man
- wenigstens einen Strom einer Suspension (4), die in einer Mutterlauge suspendierte Kristalle einer zu reinigenden Substanz enthält, am ersten Ende der Kolonne (5) mit einem Druck P2 (z.B. mittels einer Pumpe (6)), der größer als P1 ist, in selbige kontinuierliche einspeist,
- über die Filter Mutterlauge (7) in das Filterrohrinnere hinein und über die filterrohre aus der Kolonne herausführt,
- am ersten Ende der Kolonne und/oder zwischen diesem Ende und dem Filterbeginn Steuerlauge (9) in die Waschkolonne führt,
- durch die Mutter- und Steuerlaugeführung in der Kolonne ein Kristallbett (10) der zu reinigenden Substanz ausbildet, das eine dem ersten Ende der Kolonne zugewandte Aufbaufront (11) aufweist, an der sich kontinuierlich Kristalle der eingeleiteten Suspension an das Kristallbett anlagern,
- das Kristallbett durch die aus dem hydraulischen Strömungsdruckverlust der Mutter- und Steuerlaugeführung in der Kolonne resultierende Kraft an den Filtern vorbei in die zwischen den Filtern und dem zweiten Ende der Waschkolonne (12) befindliche Waschzone transportiert (13),
- an dem der Aufbaufront gegenüberliegenden Ende des Kristallbetts kontinuierlich Kristalle abträgt (14),
- die abgetragenen Kristalle aufschmilzt (15) und einen Teil der Schmelze als Waschflüssigkeitsstrom vom zweiten Ende der Kolonne herkommend gegen die Transportrichtung der Kristalle durch das Kristallbett leitet (16) und
- die Position der Aufbaufront mit Hilfe des Mengenstroms von in die Waschkolonne geführter Steuerlauge regelt,
die dadurch gekennzeichnet sind,
dass man zur Festlegung (Regelung) des Mengenstroms der in die Waschkolonne geführten Steuerlauge wenigstens einen Druckunterschied (ΔP_{SK} verwendet (heranzieht), der in der Kolonne zwischen wenigstens einem Punkt in der vor der Aufbaufront befindlichen Suspensionszone (17) und wenigstens einem Punkt in der von der Aufbaufront bis zum Ende des Kristallbetts ragenden Zone (vorzugsweise in der bis zum Beginn der Waschzone (19) ragenden Konzentrierungszone (18) des Kristallbetts) besteht.

### Beispiel

Durch fraktionierende Kondensation eines Produktgasgemisches einer zweistufigen heterogen katalysierten Gasphasenpartialoxidation von Propen wurden im Seitenabzug einer fraktionierenden Kondensationskolonne pro Stunde 1,5 t einer Rohacrylsäure der folgenden Zusammensetzungsgehalte abgezogen:

| | |
|---|---|
| Acrylsäure | 96,1 Gew.-% |
| Acrolein | 446 Gew.ppm |
| Allylacrylat | 20 Gew.ppm |
| Diacrylsäure | 3764 Gew.ppm |
| Essigsäure | 7460 Gew.ppm |
| Furfural | 6719 Gew.ppm |
| Benzaldehyd | 7131 Gew.ppm |
| Propionsäure | 751 Gew.ppm |
| Phenothiazin | 91 Gew.ppm |
| MEHQ | 247 Gew.ppm |
| Wasser | 0,83 Gew.-%. |

Durch kontinuierliche Zugabe von 22,5 kg/h Wasser zu der Rohacrylsäure wurde deren Wassergehalt auf 2,3 Gew.-% erhöht und anschließend mit einer Temperatur von 20°C einem Suspensionskristallisator zugeführt. Als Kristallisator wurde ein Kühlscheibenkristallisator (Hersteller: Firma GMF, Niederlande) mit 7 Kühlscheiben mit einem Durchmesser von 1,25 m und einem Fassungsvermögen von etwa 2500 l eingesetzt. Als Kühlmittel wurde ein Wasser/Glykol-Gemisch (70/30 Vol.-%) durch die Kühlscheiben gefahren (Eingangstemperatur = 1,5 bis 2°C). Schmelze und Kühlmittel wurden im Gegenstrom geführt. Die Schmelze wurde beim Durchgang durch den Kristallisator auf 8°C abgekühlt, wobei, bezogen auf die Suspensionsgesamtmasse, etwa 24 Gew.-% Kristalle entstanden.

Ein Teil dieser Suspension wurde kontinuierlich über eine Kreiskolbenpumpe (drehzahlgeregelt) auf eine hydraulische Waschkolonne gefahren. Diese Waschkolonne wies einen zylindrischen Prozessraum mit 263 mm Innendurchmesser auf und besaß eine den Prozessraum begrenzende Metallwand aus Edelstahl 1.4571 mit 5 mm Wandstärke. Zum Flüssigkeitsabzug wurde in der Waschkolonne ein zentral eingebautes Filterrohr (aus demselben Edelstahl) mit einem Außendurchmesser von 48 mm verwendet (Wanddicke = 2 mm). Die Länge des Prozessraums betrug 1230 mm.

Die Länge des Filterrohrs betrug 1225 mm. Die Filterlänge betrug 60 mm. Der Filter war nach einer Rohrlänge von 970 mm (von oben gemessen) eingebaut. Der Kristallabtrag am unteren Ende der Waschkolonne erfolgte mit einem rotierenden Messer (60 Umdrehungen pro Minute). Die Transportrichtung war von oben nach unten.

Die abgetragenen Kristalle wurden in einem Schmelzkreis, der bei 14°C (Schmelzpunkt der reinigend abgetrennten Kristalle) betrieben wurde, resuspendiert. Dabei wurden als Polymerisationsinhibitoren MEHQ (Monomethylether des Hydrochinons) und Luft (durch Einperlung) in die im Kreis geführte Suspension eingebracht (278 Gew.ppm MEHQ). Über einen Wärmetauscher wurde auf indirektem Weg Wärme in die im Kreis gefahrene Suspension eingetragen, um die darin resuspendierten Kristalle weitgehend aufzuschmelzen. Als Pumpe im Schmelzkreis wurde eine Kreiselpumpe (1500 U/min) mit doppeltwirkender Gleitringdichtung verwendet. Als Sperrflüssigkeit kam eine Wasser/Glykol-Mischung (85/15 Vol.-%) zum Einsatz, die indirekt mit Kühlwasser gekühlt wurde. Die Lage der Waschfront in der Kolonne wurde durch mehrere, axial auf unterschiedlicher Höhe in der Waschkolonne eingebaute, Temperaturmessungen überwacht und unter Anpassung der aus dem Schmelzkreis abgezogenen Reinproduktmenge geregelt. Die Kontrolle der Kristallbetthöhe (der Aufbaufront) erfolgte auf den bereinigten Druckunterschied ΔP_{SKB} = 250 mbar geregelt, der zwischen einer ersten offenen Bohrung innerhalb der Suspensionszone und einer zweiten offenen Bohrung 300 mm unterhalb der ersten offenen Bohrung im stationären Zustand bestand (Figur 3 zeigt einen typischen Verlauf von ΔP_{SKB} (Ordinate, mbar) in Abhängigkeit von der Entfernung (Abszisse, mm) zwischen Aufbaufront und zweiter offener Bohrung bei gleichbleibender Position der ersten offenen Bohrung). Die hydraulische Waschkolonne wurde mit einem Suspensionsmengenstrom von 1400 kg/h aus dem Kühlscheibenkristallisator beschickt. Die Temperatur der Suspension lag bei 8°C. Es stellte sich ein Überdruck gegen Atmosphäre am Kopf der Waschkolonne von 2,0 bis 2,2 bar ein, der eng begrenzt um den Mittelwert von 2,05 bar schwankte. Der Überdruck am unteren Ende der Kolonne betrug 1,8 bis 2,0 bar. Der im stationären Zustand über eine Steuerstrompumpe auf die Waschkolonne (in den Zulauf der Suspension hinter der Kreiskolbenpumpe) zurückgefahrene Steuerstrom (ein Teilstrom des über das Filter abfließenden Stoffstroms) betrug 1400 kg/h.

Der aus dem Schmelzkreis abgezogene Reinproduktstrom an gereinigter Acrylsäure lag bei 325 kg/h. Dies entspricht einer Ausbeute von 96,7 Gew.%, bezogen auf den der Waschkolonne mit der Suspension zugeführten Kristallmassenstrom. Das Reinprodukt wies folgende Zusammensetzungsgehalte auf:

| | |
|---|---|
| Acrylsäure | 99,75 Gew.%, |
| Acrolein | nicht nachweisbar, |
| Allylacrylat | nicht nachweisbar, |
| Essigsäure | 1457 Gew.-ppm |
| Furfural | 3 Gew.-ppm, |
| Benzaldehyd | 2 Gew.-ppm, |
| Propionsäure | 209 Gew.-ppm, |
| Phenothiazin | nicht nachweisbar, |
| MEHQ | 278 Gew.-ppm, |
| Wasser | < 0,05 Gew.-%. |

Waschfront und Aufbaufront waren während des gesamten Versuches von befriedigender Stabilität.

## Patentansprüche

1. Verfahren zur Regelung einer hydraulischen Waschkolonne, die eine zylindrische Einhüllende (1) aufweist, die die Kolonne begrenzt und in der sich parallel zur Zylinderachse ein oder mehrere Filterrohre (2) durch die Kolonne erstrecken, die in der Nähe des zweiten Endes der Kolonne.in der Filterrohrwand wenigstens ein Filter (3) aufweisen, das die einzige direkte Verbindung zwischen dem unter dem Druck P1 stehenden Filterrohrinneren und dem Inneren der Kolonne bildet, bei dem man
- wenigstens einen Strom einer Suspension (4), die in einer Mutterlauge suspendierte Kristalle einer zu reinigenden Substanz enthält, am ersten Ende der Kolonne (5) mit einem Druck P2 der größer als P1 ist, in selbige kontinuierlich einspeist,
- über die Filter Mutterlauge (7) in das Filterrohrinnere hinein und über die Filterrohre aus der Kolonne heraus führt,
- am ersten Ende der Kolonne und/oder zwischen diesem Ende und dem Filterbeginn gegebenenfalls Steuerlauge (9) in die Waschkolonne führt,
- durch die Mutter- und gegebenenfalls Steuerlaugeführung in der Kolonne ein Kristallbett (10) der zu reinigenden Substanz ausbildet, das eine dem ersten Ende der Kolonne zugewandte Aufbaufront (11) aufweist, an der sich kontinuierlich Kristalle der eingeleiteten Suspension ans Kristallbett anlagern,
- das Kristallbett durch die aus dem hydraulischen Strömungsdruckverlust der Mutter- und gegebenenfalls Steuerlaugeführung in der Kolonne resultierende Kraft an den Filtern vorbei in die zwischen den Filtern und dem zweiten Ende der Waschkolonne (12) befindliche Waschzone transportiert (13),
- an dem der Aufbaufront gegenüberliegenden Ende des Kristallbetts kontinuierliche Kristalle abträgt (14),
- die abgetragenen Kristalle aufschmilzt (15) und einen Teil der Schmelze als Waschflüssigkeitsstrom vom zweiten Ende der Kolonne herkommend gegen die Transportrichtung der Kristalle durch das Kristallbett leitet (16) und
- die Position der Aufbaufront mit Hilfe des Mengenstroms von in die Waschkolonne geführter Steuerlauge und/oder mit Hilfe des Mengenstroms der in die Waschkolonne geführten Suspension (4) regelt,
**dadurch gekennzeichnet**,
das man zur Festlegung (Regelung) der Mengenströme der in die Waschkolonne geführten Steuerlauge und/oder Suspension (4) wenigstens einen Druckunterschied ΔP_{SK} verwendet (heranzieht), der in der Kolonne zwischen wenigstens einem Punkt in der vor der Aufbaufront befindlichen Suspensionszone (17) und wenigstens einem Punkt in der von der Aufbaufront bis zum Ende des Kristallbetts ragenden Zone (vorzugsweise in der bis zum Beginn der Waschzone (19) ragenden Konzentrierungszone (18) des Kristallbetts) besteht.

2. Verfahren nach Anspruch 1, **dadurch gekennzeichnet, dass** man zur Festlegung der Mengenströme der in die Waschkolonne geführten Steuerlauge und/oder Suspension (4) das Verhältnis V zweier Druckunterschiede (ΔP_{SK})¹ und (ΔP_{SK})² verwendet, deren jeweilige Bezugspunkte in der von der Aufbaufront bis zum Ende des Kristallbetts ragenden Zone sich auf unterschiedlicher Höhe befinden.

3. Verfahren nach Anspruch 1, **dadurch gekennzeichnet, dass** ΔP_{SK} 50 bis 8000 mbar beträgt.

4. Verfahren nach Anspruch 2, **dadurch gekennzeichnet, dass** V 0,1 bis 0,8 beträgt.

5. Verfahren nach einem der Ansprüche 2 oder 4, **dadurch gekennzeichnet, dass** der Abstand der beiden Bezugspunkte in der von der Aufbaufront bis zum Ende des Kristallbetts ragenden Zone 10 bis 1000 mm beträgt.

6. Verfahren nach einem der Ansprüche 1 bis 5, **dadurch gekennzeichnet, dass** der jeweilige verwendete Druckunterschied ΔP_{SK} in seiner um den jeweils zugehörigen hydrostatischen Druckunterschied bereinigten Form ΔP_{SKB} verwendet wird.

7. Verfahren nach einem der Ansprüche 1 bis 6, **dadurch gekennzeichnet, dass** die im Suspensionsstrom suspendierten Kristalle Acrylsäurekristalle sind.

## Claims

1. A process for controlling a hydraulic wash column which has a cylindrical shell (1) which delimits the column and within which one or more filter tubes (2) extend through the column parallel to the cylinder axis and have, in the vicinity of the second end of the column, at least one filter (3) in the filter tube wall which forms the sole direct connection between the filter tube interior under the pressure P1 and the interior of the column in which
- at least one stream of a suspension (4) which comprises crystals of a substance to be purified suspended in a mother liquor is fed continuously into the first end of the column (5) with a pressure P2 which is greater than P1,
- mother liquor (7) is conducted through the filters into the filter tube interior and out of the column via the filter tubes,
- if appropriate, control liquor (9) is fed into the wash column at the first end of the column and/or between this end and the start of the filter,
- the mother liquor and, if appropriate, control liquor flow in the column form a crystal bed (10) of the substance to be purified, said crystal bed having a buildup front (11) which faces the first end of the column and at which crystals of the introduced suspension add continuously to the crystal bed,
- the crystal bed, by virtue of the force resulting from the hydraulic flow pressure drop of the mother liquor and, if appropriate, control liquor flow in the column, is transported (13) past the filters into the wash zone disposed between the filters and the second end of the wash column (12),
- crystals are removed (14) continuously at the opposite end of the crystal bed to the buildup front,
- the removed crystals are melted (15) and a portion of the melt is passed (16) through the crystal bed as a wash liquid stream coming from the second end of the column against the transport direction of the crystals and
- the position of the buildup front is controlled with the aid of the flow rate of control liquor conducted into the wash column and/or with the aid of the flow rate of the suspension (4) conducted into the wash column,
wherein
the flow rates of the control liquor and/or suspension (4) conducted into the wash column are determined (regulated) by utilising at least one pressure difference ΔP_{SK} which exists in the column between at least one point in the suspension zone (17) disposed in front of the buildup front and at least one point in the zone projecting from the buildup front up to the end of the crystal bed (preferably in the concentration zone (18), projecting up to the start of the wash zone (19), of the crystal bed).

2. The process according to claim 1, wherein the flow rates of control liquor and/or suspension (4) fed into the wash column are determined by using the ratio V of two pressure differences (ΔP_{SK})¹ and (ΔP_{SK})² whose particular reference points are disposed at different heights in the zone projecting from the buildup front up to the end of the crystal bed.

3. The process according to claim 1, wherein ΔP_{SK} is from 50 to 8000 mbar.

4. The process according to claim 2, wherein V is from 0.1 to 0.8.

5. The process according to either of claims 2 and 4, wherein the distance of the two reference points within the zone projecting from the buildup front up to the end of the crystal bed is from 10 to 1000 mm.

6. The process according to any of claims 1 to 5, wherein the particular pressure difference ΔP_{SK} used is used in its form ΔP_{SKB} adjusted by the particular accompanying hydrostatic pressure difference.

7. The process according to any of claims 1 to 6, wherein the crystals suspended in the suspension stream are acrylic acid crystals.

## Revendications

1. Procédé de régulation d'une colonne de lavage hydraulique présentant une extrémité enveloppante cylindrique (1), qui délimite la colonne et dans laquelle s'étendent, à travers la colonne parallèlement à l'axe du cylindre, un ou plusieurs tubes filtrants (2), qui présentent au voisinage de la deuxième extrémité de la colonne, dans la paroi du tube filtrant, au moins un filtre (3), qui forme la seule liaison directe entre l'intérieur du tube filtrant, qui se trouve sous une pression P1, et l'intérieur de la colonne, dans lequel :
- on introduit au moins un courant d'une suspension (4), qui contient, en suspension dans une eau mère, des cristaux d'une substance à purifier, à la première extrémité de la colonne (5) sous une pression P2 supérieure à P1, en continu,
- on fait passer l'eau mère (7) par les filtres dans l'intérieur du tube filtrant et hors de la colonne via les tubes filtrants,
- on introduit le cas échéant dans la colonne de lavage, à la première extrémité de la colonne et/ou entre cette extrémité et le début du filtre, un liquide d'entraînement (9),
- l'amenée de l'eau mère et le cas échéant celle du liquide d'entraînement dans la colonne forme un lit de cristaux (10) de la substance à purifier, qui présente un front d'accumulation (11) faisant face à la première extrémité de la colonne, et sur lequel se déposent continuellement des cristaux de la suspension introduite sur le lit de cristaux,
- le lit de cristaux, par suite de la force résultant de la perte de pression hydraulique de l'amenée de l'eau mère et le cas échéant de celle du liquide d'entraînement dans la colonne, est transporté (13) à travers les filtres dans la zone de lavage se trouvant entre les filtres et la deuxième extrémité de la colonne de lavage (12),
- des cristaux sont déchargés (14) en continu à l'extrémité du lit de cristaux opposée au front d'accumulation,
- les cristaux déchargés sont fondus (15) et une partie de la masse fondue est guidée (16) à travers le lit de cristaux en tant que courant liquide de lavage provenant de la deuxième extrémité de la colonne dans la direction inverse du transport des cristaux, et
- la position du front d'accumulation est régulée à l'aide du débit quantitatif du liquide d'entraînement introduite dans la colonne de lavage et/ou à l'aide du débit quantitatif de la suspension (4) introduite dans la colonne de lavage,
**caractérisé en ce que** :
pour la détermination (régulation) des débits quantitatifs du liquide d'entraînement et/ou de la suspension (4) introduits dans la colonne de lavage, on utilise au moins une différence de pression ΔP_{SK} qui existe dans la colonne entre au moins un point de la zone de suspension (17) se trouvant devant le front d'accumulation et au moins un point situé dans la zone allant du front d'accumulation à la fin du lit de cristaux (de préférence dans la zone de concentration (18) allant jusqu'au début de la zone de lavage (19) du lit de cristaux).

2. Procédé suivant la revendication 1, **caractérisé en ce que**, pour déterminer les débits quantitatifs du liquide d'entraînement et/ou de la suspension (4) introduits dans la colonne de lavage, on utilise le rapport V de deux différences de pression (ΔP_{SK})¹ et (ΔP_{SK})², dont les points de référence respectifs se trouvent à des hauteurs différentes dans la zone allant du front d'accumulation à la fin du lit de cristaux.

3. Procédé suivant la revendication 1, **caractérisé en ce que** ΔP_{SK} est de 50 à 8000 mbars.

4. Procédé suivant la revendication 2, **caractérisé en ce que** V vaut de 0,1 à 0,8.

5. Procédé suivant l'une des revendications 2 ou 4, **caractérisé en ce que** la distance des deux points de référence dans la zone allant du front d'accumulation à la fin du lit de cristaux est de 10 à 1000 mm.

6. Procédé suivant l'une quelconque des revendications 1 à 5, **caractérisé en ce que** la différence de pression respective ΔP_{SK} utilisée est utilisée sous sa forme ΔP_{SKB} corrigée de la différence de pression hydrostatique correspondante respective.

7. Procédé suivant l'une quelconque des revendications 1 à 6, **caractérisé en ce que** les cristaux en suspension dans le courant de suspension sont des cristaux d'acide acrylique.
